# EUROPEAN PATENT SPECIFICATION

(11) **EP 3 636 292 B1**
(45) Date of publication and mention of the grant of the patent: **01.03.2023**
(21) Application number: 18809500.4
(22) Date of filing: 09.05.2018
(51) Int. Cl.: A61L 27/18, A61F 2/38, A61L 27/44, A61L 27/50, A61L 27/56, A61L 27/58

(54) **MENISCUS REGENERATION SUBSTRATE**
MENISKUSREGENERIERUNGSSUBSTRAT
SUBSTRAT DE RÉGÉNÉRATION DU MÉNISQUE

(30) Priority: 29.05.2017 JP 2017105532
(43) Date of publication of application: 15.04.2020
(73) Proprietor: Educational Foundation Of Osaka Medical And Pharmaceutical University, Takatsuki-shi, Osaka 569-8686 (JP); Gunze Limited, Ayabe-shi, Kyoto 623-8511 (JP)
(72) Inventor: OTSUKI, Shuhei, Takatsuki-shi Osaka 569-8686 (JP); INOUE, Tae, Ayabe-shi Kyoto 623-8512 (JP); SEZAKI, Shunsuke, Ayabe-shi Kyoto 623-8512 (JP)
(74) Representative: Adam, Holger
(86) International application number: PCT/JP2018/017911
(87) International publication number: WO 2018/221140

(56) References cited:
- JP-A- H0 576 586
- JP-A- 2003 512 896
- JP-A- 2005 305 163
- JP-A- 2006 334 430
- JP-A- 2014 183 896
- US-A1- 2002 119 177
- US-A1- 2005 232 967
- Ootsuki, Shuhei et al.: "Research on transplant of novel semilunar valve scaffold using mini pig", Joskas, vol. 42, no. 4, P2-71-6, 2 May 2017 (2017-05-02), page 675,
- Otsuki Et Al.: "Evaluation of Meniscal Regeneration in a Mini PIG Model Treated with a Novel Polyglycolic AcId Meniscal Scaffold", The American Journal of Sports Medicine, 1 January 2019 (2019-01-01), XP55958780, DOI: 10.1177/0363546519850578 Retrieved from the Internet: URL:https://pubmed.ncbi.nlm.nih.gov/311727 97/ [retrieved on 2022-09-07]

## Description

### TECHNICAL FIELD

The present invention relates to a meniscus regeneration substrate capable of promoting meniscus regeneration when used to fill a defect of the meniscus of the knee joint in meniscus regeneration.

### BACKGROUND ART

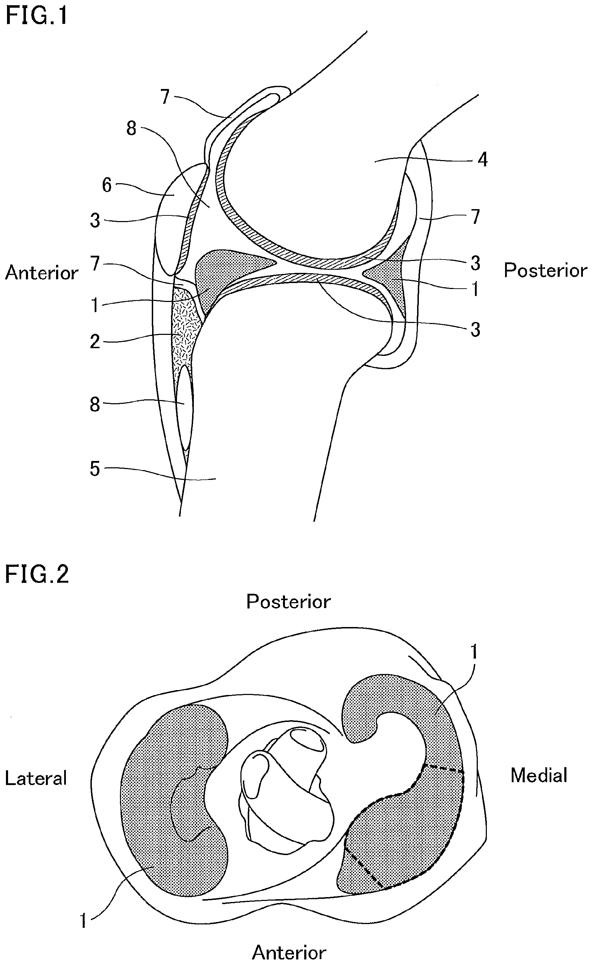

The meniscus is a cartilage-like tissue in the knee joint. The structure of the knee joint is described below with reference to Figs. 1 and 2. Fig. 1 is a schematic cross-sectional view of the right knee joint in the sagittal plane. Fig. 2 is a schematic cross-sectional view of the right knee joint in the transverse plane. As shown in Fig. 1, the knee joint has menisci 1 between a femur 4 and a tibia 5. Cartilage 3 is on the facing sides of the femur 4 and the tibia 5. A patella 6 sits on the anterior surface of the knee, and an infrapatellar fat pad (IPFP) 2 lies below the patella. The knee joint is surrounded by a joint capsule 7. The inside of the joint is filled with synovial fluid 8. As shown in Fig. 2, the menisci 1 are formed as a pair, facing each other on the medial and lateral sides of the knee joint. The menisci 1 are thicker at the anterior side and the posterior side of the knee joint.

Degeneration or damage of the meniscus is one of the conditions that are frequently observed with cartilage degeneration in osteoarthritis (OA). Some reports state that meniscectomies reduce the cartilage tissue, leading to development of osteoarthritis. The meniscus is a tissue containing a large avascular area, and thus has poor self-regenerative capacity and hardly self-repairs. To promote healing of the meniscus, surgeries have included, besides meniscus suture, additional treatments such as injection of growth factors, synovial transplantation, and bone marrow stimulation. Still, regeneration of the meniscus is insufficient.

Meanwhile, recent advances in the cell engineering have enabled culturing of various animal cells, including human cells. Research on the reconstruction of human tissues or organs using such cells, that is, what is called regenerative medicine, is progressing rapidly. The point of regenerative medicine is whether cells can grow and differentiate into a three-dimensional, living tissue-like structure. In an exemplary method, a substrate is implanted into the patient's body so that cells from the surrounding tissue or organ can enter the substrate and grow and differentiate to regenerate tissue or an organ.

Substrates for regenerative medicine that have been proposed include non-woven fabrics containing bioabsorbable materials, such as one disclosed in Patent Literature 1. When a non-woven fabric containing a bioabsorbable material is used as a substrate for regenerative medicine or as a suture reinforcement material, cells enter its voids and grow, allowing rapid tissue regeneration. Use of such tissue regeneration substrates has been started also in meniscus regeneration.

Patent Literature 2 provides a biocompatible meniscal device comprising a biocompatible meniscal repair device comprising a biocompatible tissue repair scaffold adapted to be placed in contact with a defect in a meniscus. The scaffold is formed from a nonwoven material and a biocompatible foam. In one embodiment, the foam material is formed as a layer on both sides of a layer of nonwoven material.The nonwoven material of the scaffold is formed from one or more biocompatible polymers. The thickness of the scaffold is preferably in the range of 0.5 to 1.5mm . A lactide-epsilon-caprolactone can be used as the foaming material . The density of the non-woven can be up to 360mg/cc.

In actual, however, conventional tissue regeneration substrates do not promote meniscus regeneration as much as expected.

### CITATION LIST

### - Patent Literature

Patent Literature 1: JP H05-076586 A
Patent Literature 2: US 2005/232967

### SUMMARY OF INVENTION

### - Technical problem

The present invention aims to provide a meniscus regeneration substrate capable of promoting meniscus regeneration when used to fill a defect of the meniscus of the knee joint in meniscus regeneration.

### - Solution to problem

The present invention relates to a meniscus regeneration substrate capable of promoting meniscus regeneration when used to fill a defect of a meniscus of a knee joint in meniscus according to claim 1. The present invention is described in detail below.

The present inventors studied the reason why conventional tissue regeneration substrates fail to sufficiently promote the meniscus regeneration. The inventors found out that while conventional tissue regeneration substrates give good results in animal tests using small animals such as leporine, they fail to sufficiently regenerate the meniscus in large animals such as porcine or bovine. The inventors made intensive studies to find out that insufficient physical strength of the conventional tissue regeneration substrates is the reason for unsatisfactory results with large animals.

The knee joints support all the body weight during standing or walking. The impact is significant particularly in large animals including human. Even if the recipient tries to get as much rest as possible after implantation, the implanted meniscus regeneration substrate keeps receiving great stress during the long healing process. Moreover, meniscus regeneration has its unique problem. That is, the implanted substrate comes in contact with cartilage due to movement of the knee joint, causing friction that wears down the surface of the substrate.

It is known that the non-woven fabrics constituting the conventional tissue regeneration substrates preferably have an average pore size of about 5 to 30 µm to ensure the permeability to cells. However, such conventional tissue regeneration substrates, although excellent in permeability to cells, do not have sufficient mechanical strength. Such substrates thus become thinner due to damage or wearing down of the surface before the meniscus regenerates, and assumably eventually become unable to function as a scaffold to regenerate a meniscus with a sufficient thickness.

After further intensive studies, the present inventors arrived at a laminate that has a certain thickness or more and includes a sponge layer containing a bioabsorbable material having a lower decomposition rate than polyglycolide (hereinafter also referred to simply as a "sponge layer") on each of the upper and lower surfaces (both surfaces) of a non-woven fabric layer containing polyglycolide and having a 50% compression stress of 0.5 MPa (hereinafter also referred to simply as a "non-woven fabric layer"). The inventors found out that such a laminate can maintain a sufficient thickness without suffering damage or wearing down of the surfaces even when undergoing great stress after implantation, and thus can regenerate a meniscus with a sufficient thickness. The inventors thus completed the present invention.

The meniscus regeneration substrate of the present invention has a structure in which the sponge layer, the non-woven fabric layer, and the sponge layer are integrated in the stated order.

The non-woven fabric layer serves as a scaffold for the growth of cells entering from the surrounding tissues after implantation. The non-woven fabric layer functions to promote meniscus regeneration, as well as to exhibit sufficient mechanical strength when the meniscus regeneration substrate undergoes stress after implantation.

The non-woven fabric layer has a 50% compression stress of 0.5 MPa or higher. The non-woven fabric layer having a 50% compression stress of 0.5 MPa or higher allows regeneration of a meniscus with a sufficient thickness without damage to the meniscus regeneration substrate even when the substrate undergoes great stress after implantation. The 50% compression stress of the non-woven fabric layer is preferably 0.8 MPa or higher, more preferably 1.0 MPa or higher. The upper limit of the 50% compression stress of the non-woven fabric layer is not limited. To further promote the regeneration of the meniscus tissue, the upper limit is preferably 8.0 MPa.

The 50% compression stress can be measured by a method in accordance with JIS-K6767.

When the non-woven fabric layer has a density of 300 mg/cm³ or higher, the 50% compression stress of the non-woven fabric layer can be easily adjusted to 0.5 MPa or higher. The density of the non-woven fabric layer is 400 mg/cm³ or higher. The upper limit of the density of the non-woven fabric layer is not limited. To further promote regeneration of the meniscus tissue, the upper limit is preferably 500 mg/cm³.

The non-woven fabric constituting the non-woven fabric layer has an average fiber size. The lower limit thereof is 25 denier and the upper limit thereof is 40 denier. When the average fiber size of the non-woven fabric constituting the non-woven fabric layer is within this range, the 50% compression stress of the non-woven fabric layer can be easily adjusted to 0.5 MPa or higher. The lower limit of the average fiber size of the non-woven fabric constituting the non-woven fabric layer is more preferably 30 denier and the upper limit thereof is more preferably 32 denier.

The non-woven fabric layer has a thickness of 2 mm or more. The non-woven fabric layer having a thickness of 2 mm or more allows regeneration of a meniscus with a sufficient thickness. The thickness of the non-woven fabric layer is preferably 4 mm or more. The upper limit of the thickness of the non-woven fabric is not limited, but is practically 10 mm from the standpoint of applications.

The non-woven fabric layer contains polyglycolide.

As described above, the non-woven fabric layer has a 50% compression stress of 0.5 MPa or higher. To achieve such a high 50% compression stress, the non-woven fabric layer needs to have a very dense structure. According to the conventional common technical knowledge, the non-woven fabric constituting the tissue regeneration substrate preferably has an average pore size of about 5 to 30 µm to ensure permeability to cells. Such a low density non-woven fabric, however, cannot possibly achieve a 50% compression stress of 0.5 MPa or higher. Surprisingly, the non-woven fabric layer containing polyglycolide allows cells to enter the layer and sufficiently regenerate the meniscus even when the layer has a very dense structure with a 50% compression stress of 0.5 MPa or higher which has been considered to be difficult for cells to enter.

When polyglycolide fibers are immersed in 37°C saline, it takes about 14 days for the tensile strength of the fibers to fall to half, of its value before immersion. Such decomposability allows cells to enter the non-woven fabric layer and grow as the decomposition and absorption proceed, despite the very dense structure with a 50% compression stress of 0.5 MPa or higher. The cells construct regenerated tissue to the inside the non-woven fabric layer, which is considered to result in the construction of a good-quality regenerated tissue. Furthermore, since inflammatory cells disappear within several days after implantation in the living body, the non-woven fabric layer containing polyglycolide also exhibits the advantageous effect that it less likely to cause tissue adhesions.

The polyglycolide as used herein means a polymer of glycolide such as polyglycolic acid, but may be a copolymer with other bioabsorbable component(s) such as lactide, ε-caprolactone, or p-dioxanone as long as the effects of the present invention are not inhibited. The polyglycolide may be mixed with other bioabsorbable material(s) such as polylactide as long as the effects of the present invention are not inhibited.

When the polyglycolide is a copolymer with other bioabsorbable component(s) such as lactide, ε-caprolactone, or p-dioxanone, the lower limit of the amount of the glycolide component in the copolymer is preferably 60 mol%. When the amount of the glycolide component is 60 mol% or more, the advantageous effects of the present invention, that is, excellent permeability to cells and regeneration of a normal tissue, can be particularly exhibited.

When a mixture of the polyglycolide and other bioabsorbable material(s) such as polylactide is used, the lower limit of the amount of the polyglycolide in the mixture is preferably 50 mol%. When the amount of the polyglycolide is 50 mol% or more, the advantageous effects of the present invention, that is, excellent permeability to cells and regeneration of a normal tissue, can be particularly exhibited.

The lower limit of the weight average molecular weight of the polyglycolide is preferably 30000 and the upper limit thereof is preferably 600000. The polyglycolide having a weight average molecular weight of less than 30000 may have insufficient strength and fail to provide a 50% compression stress of 0.5 MPa or higher. The polyglycolide having a weight average molecular weight of more than 600000 may have a low decomposition rate in the living body and cause foreign-body reaction. The lower limit of the weight average molecular weight of the polyglycolide is more preferably 50000 and the upper limit thereof is more preferably 400000.

The melt flow rate may be used as an alternative index to the molecular weight of the polyglycolide. The lower limit of the melt flow rate of the polyglycolide is preferably 0.1 g/10 min and the upper limit thereof is preferably 100 g/10 min. When the melt flow rate is within the range, the non-woven fabric containing the polyglycolide can be easily produced. The lower limit of the melt flow rate of the polyglycolide is more preferably 1 g/10 min and the upper limit thereof is more preferably 50 g/10 min.

The melt flow rate means a value measured at a load of 4 kgf after melting the polyglycolide by holding it at 240°C for 10 minutes in a cylinder.

The non-woven fabric layer may be prepared by any method, and may be prepared by a conventionally known method such as an electro-spinning deposition method, a melt blow method, a needle punching method, a spun-bonding method, a flash spinning method, a spun-lacing method, an airlaid method, a thermal bonding method, a resin bonding method, or a wet method.

Here, with a non-woven fabric produced by a conventional method, it may be difficult to obtain a non-woven fabric layer having a 50% compression stress of 0.5 MPa or higher. In such a case, multiple sheets of the non-woven fabric may be stacked to be integrated, and compressed by a method such as hot press to adjust the 50% compression stress to 0.5 MPa or higher.

The compressed non-woven fabric sheets are further sewn with a sewing machine using yarn containing a bioabsorbable material, as needed. The compressed non-woven fabric sheets undergo a stress that tries to restore the original thickness. Sewing with a sewing machine to fix the thickness can prevent a reduction in the density. The pitch in the sewing with a sewing machine is not limited. To reliably fix the thickness, the pitch is preferably 5 mm or less.

The sponge layers each contain a bioabsorbable material having a lower decomposition rate than polyglycolide. Interposing the non-woven fabric layer between the sponge layers each containing a bioabsorbable material having a lower decomposition rate than polyglycolide can protect the non-woven fabric layer until cells enter the non-woven fabric layer and sufficiently regenerate the meniscus.

Any bioabsorbable material having a lower decomposition rate than polyglycolide may be used.
Examples thereof include polylactide (D, L, or DL isomer), polycaprolactone, glycolide-lactide (D, L, or DL isomer) copolymers, glycolide-ε-caprolactone copolymers, lactide (D, L, or DL isomer)-ε-caprolactone copolymers, polydioxanone, and glycolide-lactide (D, L, or DL isomer)-ε-caprolactone copolymers. Preferred among them are lactide (D, L, or DL isomer)-ε-caprolactone copolymers, which have a decomposition rate sufficiently lower than polyglycolide and have excellent flexibility and abrasion resistance. Interposing the non-woven fabric layer between sponge layers containing a lactide (D, L, or DL isomer)-ε-caprolactone copolymer can impart appropriate flexibility to the meniscus regeneration substrate of the present invention, and also prevent the surface of the substrate from wearing down when the substrate comes in contact with cartilage due to movement of the knee joint after implantation.

The sponge layers may each have any density. The lower limit of the density is preferably 40 mg/cm³ and the upper limit thereof is preferably 120 mg/cm³. When the density of each sponge layer is within this range, sufficient flexibility and abrasion resistance can be exhibited. The lower limit of the density of each sponge layer is more preferably 70 mg/cm³ and the upper limit thereof is more preferably 90 mg/cm³.

The sponge layers may each have any thickness. The lower limit thereof is preferably 0.1 mm and the upper limit thereof is preferably 1.0 mm. The sponge layers having a thickness within this range can sufficiently function to prevent the surface of the substrate from wearing down when the substrate comes in contact with cartilage due to movement of the knee joint after implantation.

The method for preparing the sponge layers is not limited. The sponge layers may be produced by a conventionally known method such as one in which a solution of the bioabsorbable material in an appropriate solvent is frozen and then freeze-dried.

To impart slipperiness and thereby further exhibit abrasion resistance, the meniscus regeneration substrate of the present invention may include, as an outermost layer, a film layer containing a bioabsorbable material having a lower decomposition rate than polyglycolide on one or both surfaces of the laminate including the sponge layer, the non-woven fabric layer, and the sponge layer.

The film as used herein means a thin sheet that is at least free of pores on the order of micrometers observable with an optical microscope.

The film layer may contain any bioabsorbable material having a lower decomposition rate than polyglycolide. Suitable examples thereof include lactide (D, L, or DL isomer)-ε-caprolactone copolymers.

The film layer may contain a lubricant to further improve the slipperiness without impairing a medical use.

The film layer may have any thickness. The lower limit thereof is preferably 0.1 mm and the upper limit thereof is preferably 0.3 mm. The film layer having a thickness of less than 0.1 mm may not sufficiently prevent the surface of the substrate from wearing down when the substrate comes in contact with cartilage due to movement of the knee joint after implantation. The film layer having a thickness of more than 0.3 mm may have poor handleability.

The sponge layers and the non-woven fabric layer (and the film layer) are integrated.

If the layers are not integrated, delamination may occur at part or all of the interfaces between the layers in the implantation of the meniscus regeneration substrate of the present invention. Even delamination at only part of the interfaces may cause build-up of cells in the space formed in the delamination portion, which may prevent regeneration of a normal tissue or organ.

The "integrated" as used herein means that the layers are laminated with enough adhesive force that the sponge layers and the non-woven fabric layer (and the film layer) cannot be separated from one another by simply pulling with the hands.

The meniscus regeneration substrate of the present invention preferably has a thickness retention of 95% or higher after a repeated compression test that includes repeating, 20 times, the application of a load of 300 N to the meniscus regeneration substrate having a size of 20 mm in length and 20 mm in width in warm water at a temperature of 37°C. Such a meniscus regeneration substrate can maintain a sufficient thickness even when the substrate repeatedly undergoes great stress after implantation, allowing regeneration of a meniscus with a sufficient thickness. The thickness retention is more preferably 98% or more, still more preferably 100%.

The thickness retention means that the ratio (%) of the thickness of the meniscus regeneration substrate after the repeated compression test to the thickness of the meniscus regeneration substrate before the repeated compression test.

The meniscus regeneration substrate of the present invention may be produced by any method. For example, the non-woven fabric layer and the sponge layers (and the film layer) may be separately prepared, and then bonded together with a medical adhesive or bonded after part of a surface of each layer is dissolved with a solvent.

### - Advantageous Effects of Invention

The present invention can provide a meniscus regeneration substrate capable of promoting meniscus regeneration when used to fill a defect of the meniscus of the knee joint in meniscus regeneration.

### BRIEF DESCRIPTION OF DRAWINGS

Fig. 1 is a schematic cross-sectional view of the right knee joint in the sagittal plane.
Fig. 2 is a schematic cross-sectional view of the right knee joint in the transverse plane.
Fig. 3 shows magnetic resonance images (MRIs) taken 0, 4, and 8 weeks after implantation of a meniscus regeneration substrate obtained in Example 1 in a meniscus removal portion in a pig.
Fig. 4 is an image of an implanted portion stained with hematoxylin and eosin in the 8th week after implantation of the meniscus regeneration substrate obtained in Example 1 in a meniscus removal portion in a pig <Animal test evaluation 1>.
Fig. 5 is an image of an implanted portion stained with safranin in the 8th week after implantation of the meniscus regeneration substrate obtained in Example 1 into a meniscus removal portion in a pig <Animal test evaluation 1>.
Fig. 6 is an image of the entire menisci taken out in the 4th week after implantation of the meniscus regeneration substrate obtained in Example 1 in a meniscus removal portion in a pig <Animal test evaluation 2>.
Fig. 7 is an image of the entire menisci taken out in the 8th week after implantation of the meniscus regeneration substrate obtained in Example 1 in a meniscus removal portion in a pig <Animal test evaluation 2>.
Fig. 8 is an image of the entire meniscus taken out in the 12th week after implantation of the menisci regeneration substrate obtained in Example 1 in a meniscus removal portion in a pig <Animal test evaluation 2>.

### DESCRIPTION OF EMBODIMENTS

Embodiments of the present invention are described in more detail below with reference to examples. The present invention, however, should not be limited to these examples.

### (Example 1)

### (1) Preparation of non-woven fabric layer

Green-stained polyglycolide having a weight average molecular weight of 250000 was used as a bioabsorbable material. The polyglycolide was spun into yarn having an average fiber size of 31 denier, and a fabric made of the yarn was formed into a non-woven fabric by a needle punching method. In this manner, a green-stained non-woven fabric having an average fiber size of about 20 µm and a thickness of about 300 µm was obtained.

Fourteen sheets of the obtained non-woven fabric were stacked and needle punched from one side to be integrated. The integrated stack was hot pressed at a temperature of 100°C for one minute and sewn with a sewing machine at a pitch of 5 mm using polyglycolide yarn of 400 denier. Thus a non-woven fabric layer having a thickness of 3.7 mm was obtained.

The 50% compression stress of the obtained non-woven fabric layer was 3.5 MPa as measured by a method in accordance with JIS-K6767. The density of the obtained non-woven fabric layer was 400 mg/cm³.

### (2) Preparation of sponge layers

A L-lactide-ε-caprolactone copolymer (molar ratio: 50:50, weight average molecular weight: 200000) was dissolved in dioxane to give a 4% by weight solution in dioxane. The non-woven fabric layer was left in a glass petri dish. The obtained solution was poured into the glass petri dish such that the level of the solution was substantially equal to the height of the non-woven fabric layer. The solution was frozen at -80°C for one hour and then freeze-dried at -40°C for 12 hours using a freeze-dryer (available from ADVANTEC, DRZ350WA) to form sponge layers, each having a thickness of about 100 µm, on both surfaces of the non-woven fabric layer. Thus, a laminate in which the sponge layer, the non-woven fabric layer, and the sponge layer were integrally laminated was obtained.

### (3) Production of meniscus regeneration substrate

A L-lactide-ε-caprolactone copolymer (molar ratio: 50:50, weight average molecular weight: 400000) was dissolved in dioxane to give a 4% by weight solution in dioxane. The obtained solution was poured into a glass petri dish, and air-dried and heat-treated. In this manner, films each having a thickness of about 100 µm were obtained.

A small amount of dioxane was applied to one surface of each obtained film to dissolve part of the surface. The films were stacked on the laminate in which the sponge layer, the non-woven fabric layer, and the sponge layer were integrally laminated, such that the films came into contact with the sponge layers on the upper and lower surfaces (both surfaces) of the laminate. The films were dried to be integrated with the laminate, whereby film layers each having a thickness of about 100 µm were formed. Thus, a meniscus regeneration substrate was obtained in which the film layer, the sponge layer, the non-woven fabric layer, the sponge layer, and the film layer were integrally laminated. The 50% compression stress of the obtained meniscus regeneration substrate was 3.8 MPa as measured by a method in accordance with JIS-K6767.

The obtained meniscus regeneration substrate was cut to a size of 20 mm in length and 20 mm in width to prepare a sample. The obtained sample was put in warm water at a temperature of 37°C and subjected to a repeated compression test including repeating the application of a load of 300 N to the sample 20 times. The thickness of the meniscus regeneration substrate was measured before and after the repeated compression test. The thickness retention was calculated to be 100%.

### <Animal test evaluation 1>

A pig, which is a large animal, was provided as a test animal. The anterior segment of the medial meniscus of the right knee joint was removed. The meniscus regeneration substrate obtained in Example 1 was implanted in the meniscus removal portion, followed by suturing.

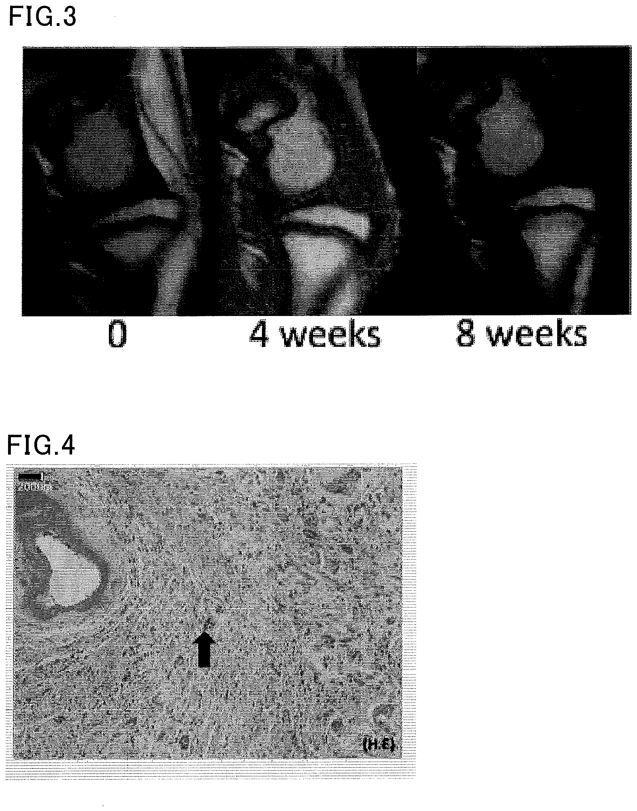

Magnetic resonance images (MRIs) of the right knee joint were taken 0, 4, and 8 weeks after the operation. Fig. 3 shows the magnetic resonance images (MRIs).

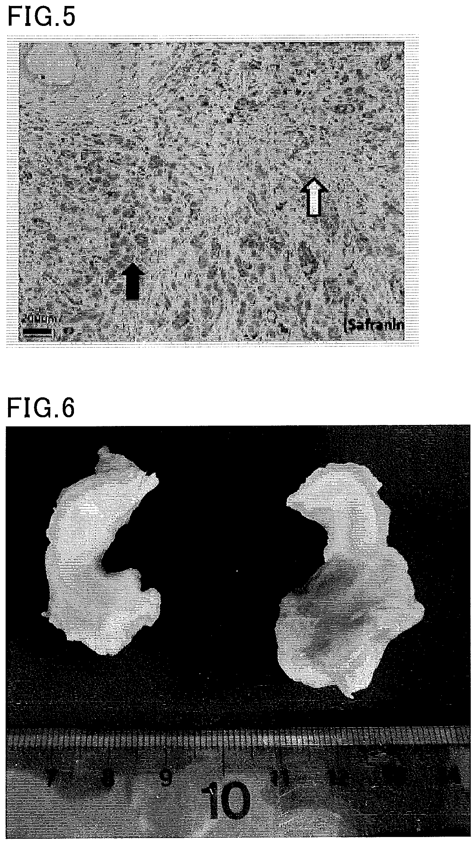

The pig was sacrificed in the 8th postoperative week. The medial meniscus of the right knee joint was taken out, and the implanted portion was extracted. Fig. 4 shows a micrograph of the obtained sample stained with hematoxylin and eosin. Fig. 5 shows a micrograph of the obtained sample stained with safranin.

Fig. 3 shows that cartilage was regenerated first from the contact portion of the joint. Moreover, Fig. 4 and Fig. 5 show that in the 8th postoperative week, cartilage tissue (red-stained portions in the stained image in Fig. 5) was regenerated in the portion where the meniscus regeneration substrate was implanted.

### <Animal test evaluation 2>

A pig, which is a large animal, was provided as a test animal. The anterior segment of the medial meniscus of the right knee joint was removed. The meniscus regeneration substrate obtained in Example 1 was implanted in the meniscus removal portion, followed by suturing. The implantation was performed in three pigs.

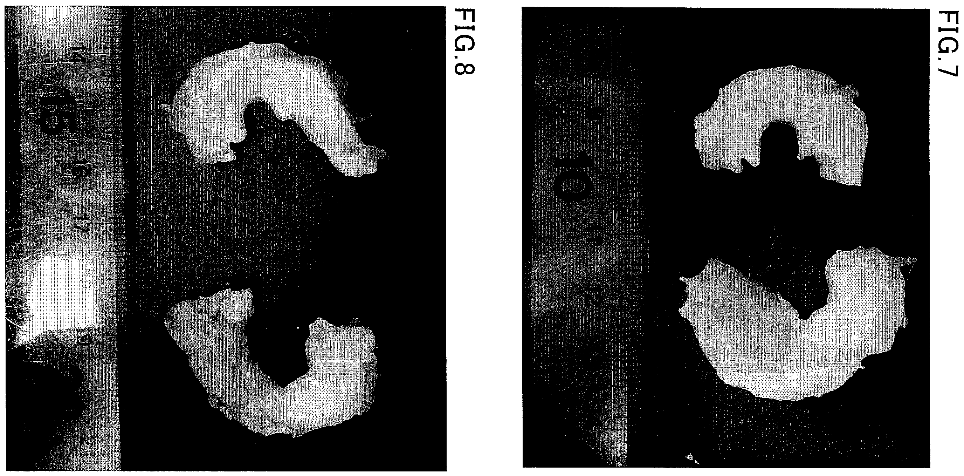

The pigs were sacrificed, one in each of the 4th, 8th, and 12th postoperative weeks. The medial meniscus of the right knee joint was taken out, and an image of the entire meniscus was captured. The lateral meniscus of the right knee joint not implanted with the meniscus regeneration substrate was also taken out as a control, and an image thereof was captured. Figs. 6 to 8 show the images. In each figure, on the left is the control, and on the right is the meniscus implanted with the meniscus regeneration substrate.

Fig. 6 shows the green-colored non-woven fabric layer remaining in the implanted portion of the meniscus regeneration substrate in the 4th postoperative week. In the 8th and 12th postoperative weeks, the non-woven fabric layer hardly remained, while gradual regeneration of the meniscus tissue was observed.

### INDUSTRIAL APPLICABILITY

The present invention can provide a meniscus regeneration substrate capable of promoting meniscus regeneration when used to fill a defect of the meniscus of the knee joint in meniscus regeneration.

### REFERENCE SIGNS LIST

1 meniscus
2 infrapatellar fat pad
3 cartilage
4 femur
5 tibia
6 patella
7 joint capsule
8 synovial fluid

## Claims

1. A meniscus regeneration substrate capable of promoting meniscus regeneration when used to fill a defect of a meniscus of a knee joint in meniscus regeneration,
the meniscus regeneration substrate having a structure in which a sponge layer containing a bioabsorbable material having a lower decomposition rate than polyglycolide, a non-woven fabric layer containing polyglycolide, and a sponge layer containing a bioabsorbable material having a lower decomposition rate than polyglycolide are integrated in the stated order,
the non-woven fabric layer having a 50% compression stress of 0.5 MPa or higher and a thickness of 2 mm or more,
wherein the non-woven fabric layer has a density of 400 mg/cm³ or higher,
wherein the non-woven fabric layer contains a non-woven fabric having an average fiber size from 25 denier to 40 denier,
wherein the non-woven fabric layer is sewn with a sewing machine using yarn containing a bioabsorbable material, and the pitch in the sewing with a sewing machine is 5 mm or less.

2. The meniscus regeneration substrate according to claim 1,
wherein each bioabsorbable material having a lower decomposition rate than polyglycolide is a lactide-ε-caprolactone copolymer.

3. The meniscus regeneration substrate according to claim 1 or 2, comprising, on one or both surfaces thereof, a film layer containing a bioabsorbable material having a lower decomposition rate than polyglycolide.

4. The meniscus regeneration substrate according to claim 1, 2 or 3,
which has a thickness retention of 95% or higher after a repeated compression test that includes repeating, 20 times, application of a load of 300 N to the meniscus regeneration substrate having a size of 20 mm in length and 20 mm in width in warm water at a temperature of 37°C.

## Patentansprüche

1. Meniskusregenerierungssubstrat, das in der Lage ist, die Meniskusregeneration zu fördern, wenn es dazu verwendet wird, einen Defekt eines Meniskus eines Kniegelenks bei der Meniskusregeneration zu füllen,
wobei das Meniskusregenerierungssubstrat eine Struktur aufweist, in der eine Schwammschicht, die ein bioabsorbierbares Material mit einer geringeren Zersetzungsrate als Polyglycolid enthält, eine Vliesstoffschicht, die Polyglycolid enthält, und eine Schwammschicht, die ein bioabsorbierbares Material mit einer geringeren Zersetzungsrate als Polyglycolid enthält, in der angegebenen Reihenfolge integriert sind,
wobei die Vliesstoffschicht eine 50%ige Druckspannung von 0,5 MPa oder mehr und eine Dicke von 2 mm oder mehr aufweist,
wobei die Vliesstoffschicht eine Dichte von 400 mg/cm³ oder mehr aufweist,
wobei die Vliesstoffschicht einen Vliesstoff mit einer durchschnittlichen Fasergröße von 25 Denier bis 40 Denier enthält,
wobei die Vliesstoffschicht mit einer Nähmaschine unter Verwendung von Garn, das ein bioabsorbierbares Material enthält, vernäht ist und der Abstand beim Nähen mit einer Nähmaschine 5 mm oder weniger beträgt.

2. Meniskusregenerierungssubstrat nach Anspruch 1, wobei jedes bioabsorbierbare Material, das eine geringere Zersetzungsrate als Polyglycolid aufweist, ein Lactid-ε-Caprolacton-Copolymer ist.

3. Meniskusregenerierungssubstrat nach Anspruch 1 oder 2, das auf einer oder beiden Oberflächen eine Filmschicht umfasst, die ein bioresorbierbares Material mit einer geringeren Zersetzungsrate als Polyglycolid enthält.

4. Meniskusregenerierungssubstrat nach Anspruch 1, 2 oder 3,
das nach einem wiederholten Drucktest, bei dem 20-mal eine Last von 300 N auf das Meniskus-Regenerationssubstrat mit einer Größe von 20 mm Länge und 20 mm Breite in warmem Wasser bei einer Temperatur von 37°C aufgebracht wird, eine Dickenretention von 95 % oder mehr aufweist.

## Revendications

1. Substrat de régénération du ménisque capable de favoriser la régénération du ménisque lorsqu'il est utilisé pour remplir un défaut d'un ménisque d'une articulation du genou dans la régénération du ménisque,
le substrat de régénération du ménisque ayant une structure dans laquelle une couche d'éponge contenant un matériau bioabsorbable ayant une vitesse de décomposition inférieure à celle du polyglycolide, une couche de tissu non tissé contenant du polyglycolide, et une couche d'éponge contenant un matériau bioabsorbable ayant une vitesse de décomposition inférieure à celle du polyglycolide sont intégrées dans l'ordre indiqué,
la couche de tissu non tissé ayant une contrainte de compression à 50 % de 0,5 MPa ou plus et une épaisseur de 2 mm ou plus,
dans lequel la couche de tissu non tissé a une densité de 400 mg/cm³ ou plus,
dans lequel la couche de tissu non tissé contient un tissu non tissé ayant une taille de fibre moyenne de 25 deniers à 40 deniers,
dans lequel la couche de tissu non tissé est cousue avec une machine à coudre en utilisant un fil contenant un matériau bioabsorbable, et le pas dans la couture avec une machine à coudre est de 5 mm ou moins.

2. Substrat de régénération du ménisque selon la revendication 1,
dans lequel chaque matériau bioabsorbable ayant une vitesse de décomposition inférieure à celle du polyglycolide est un copolymère lactide-ε-caprolactone.

3. Substrat de régénération du ménisque selon la revendication 1 ou 2, comprenant, sur l'une ou les deux surfaces de celui-ci, une couche de film contenant un matériau bioabsorbable ayant une vitesse de décomposition inférieure à celle du polyglycolide.

4. Substrat de régénération du ménisque selon la revendication 1, 2 ou 3,
qui présente une rétention d'épaisseur de 95 % ou plus après un essai de compression répété qui comprend la répétition, 20 fois, de l'application d'une charge de 300 N au substrat de régénération du ménisque ayant une taille de 20 mm en longueur et 20 mm en largeur dans de l'eau chaude à une température de 37°C.
